# FASCICULE DE BREVET EUROPEEN

(11) **EP 3 194 940 B1**
(45) Date de publication et mention de la délivrance du brevet: **06.12.2023**
(21) Numéro de dépôt: 15763568.1
(22) Date de dépôt: 14.09.2015
(51) Int. Cl.: G01N 22/02, G01N 33/38

(54) **PROCEDE DE CONTROLE NON DESTRUCTIF POUR UNE PIECE REFRACTAIRE**
VERFAHREN ZUR ZERSTÖRUNGSFREIEN PRÜFUNG EINES FEUERFESTEN TEILS
METHOD FOR NON-DESTRUCTIVE TESTING OF A REFRACTORY PART

(30) Priorité: 19.09.2014 FR 1458851
(43) Date de publication de la demande: 26.07.2017
(73) Titulaire: SAINT-GOBAIN CENTRE DE RECHERCHES ET D'ETUDES EUROPEEN, 92400 Courbevoie (FR)
(72) Inventeur: BORIES, Olivier, F-84306 Cavaillon Cedex (FR)
(74) Mandataire: Cabinet Nony
(86) Numéro de dépôt international: PCT/EP2015/070954
(87) Numéro de publication internationale: WO 2016/041902

(56) Documents cités:
- WO-A2-99/02979
- FR-A1- 2 689 245
- Cam Nguyen ET AL: "Design concepts for a miniature pavement GPR antenna", , 1 janvier 1995 (1995-01-01), XP055171157, Extrait de l'Internet: URL:http://d2dtl5nnlpfr0r.cloudfront.net/t ti.tamu.edu/documents/1341-3F.pdf [extrait le 2015-02-20]
- B. CARROLL ET AL: "FREQUENCY-MODULATED CONTINUOUS-WAVE (FM-CW) RADAR FOR EVALUATION OF REFRACTORY STRUCTURES USED IN GLASS MANUFACTURING FURNACES", AIP CONFERENCE PROCEEDINGS, 1 janvier 2009 (2009-01-01), pages 402-409, XP055171142, DOI: 10.1063/1.3114260
- R S T - THE RADAR COMPANY: "Radar Systems Technology - Reaching Beyond Boundaries", , 1 avril 2012 (2012-04-01), XP055171174, Extrait de l'Internet: URL:http://www.rst-group.biz/uploads/media /RST_Broschuere_DE_01.pdf [extrait le 2015-02-20]
- S. KHARKOVSKY ET AL: "MICROWAVE MEASUREMENT OF REFRACTORY MATERIALS AT HIGH-TEMPERATURE", AIP CONFERENCE PROCEEDINGS, 1 janvier 2009 (2009-01-01), pages 1703-1710, XP055171108, DOI: 10.1063/1.3114164
- J Barnard: "POSSIBLE NON-DESTRUCTIVE TESTING METHOD FOR DETERMINING THE EXTENT OF HYDRATION IN MAGNESIA- BASED REFRACTORY BRICKS", , 1 janvier 2010 (2010-01-01), XP055171121, Extrait de l'Internet: URL:http://www.saimm.co.za/Conferences/Ref ractories2010/117-128_Barnard.pdf [extrait le 2015-02-20]
- JEONGWOO HAN ET AL: "Ultra-wideband electronically tunable pulse generators", IEEE MICROWAVE AND WIRELESS COMPONENTS LETTERS, vol. 14, no. 3, 1 mars 2004 (2004-03-01), pages 112-114, XP055171177, ISSN: 1531-1309, DOI: 10.1109/LMWC.2004.825184
- BERNHARD FLEISCHMANN: "Non-destructive testing of refractories, especially AZS materials, with ultrasound, microwave and gamma-radiation", GLASTECHN. BER. GLASS SCI. TECHNOL., vol. 68, no. 8, 1995, pages 259-265, XP008175072,
- Takaichi Shinya ET AL: "Application of ultrashort-pulse radar to non-destructive inspection", Communication Technology, 2008. ICCT 2008. 11th IEEE International Conference on, 1 November 2008 (2008-11-01), pages 316-318, XP055817173, Piscataway, NJ, USA DOI: 10.1109/ICCT.2008.4716255 ISBN: 978-1-4244-2250-0

## Description

### Domaine technique

L'invention se rapporte à un procédé non destructif de contrôle de la structure interne d'une pièce réfractaire.

### Etat de la technique

Les produits réfractaires ont diverses applications généralement à haute température. Ils se présentent généralement sous la forme de pièces de grandes dimensions et peuvent par exemple être utilisés dans les fours de fusion du verre.

Les pièces réfractaires peuvent notamment être produites par électrofusion. Ce procédé consiste à fondre les matières premières à très haute température dans un four à arc, puis à les couler dans un moule. La pièce moulée est ensuite solidifiée et refroidie. Durant cette étape de refroidissement, des phénomènes de retrait peuvent apparaître et créer des cavités (défauts) à l'intérieur des pièces réfractaires.

Le choix d'une composition adaptée ainsi que la maîtrise des matières premières et des procédés de fabrication permettent d'obtenir des pièces réfractaires de grande qualité adaptées aux diverses régions de four dans lesquels elles peuvent être disposées.

Une pièce réfractaire subit cependant une usure, en particulier dans certaines régions critiques très sollicitées, par exemple en contact avec la surface du bain de verre en fusion. La connaissance de sa structure interne permet de s'assurer qu'elle ne présente pas de défauts dans ces régions critiques. Des procédés de contrôle non destructif sont donc utilisés pour évaluer les défauts internes d'une pièce réfractaire :
Le procédé ultrasonique consiste à mesurer l'atténuation d'un signal ultra sonore au travers de la pièce réfractaire immergée dans de l'eau. Ce procédé est très efficace mais est le plus souvent mis en oeuvre en positionnant de chaque pièce réfractaire dans une cuve remplie d'eau.

Par ailleurs, FR 2 689 245 divulgue l'utilisation de la technologie des radars à pénétration de sol (RPS) (en anglais « GPR » pour « Ground Penetrating Radar »).

Cependant, il existe une zone aveugle, située directement sous les antennes du radar, qui représente plusieurs dizaines de millimètres et dans laquelle il est impossible de détecter d'éventuels défauts. Ce procédé est donc inefficace pour toutes les pièces réfractaires de faible épaisseur (par exemple les dalles) car la zone de défauts potentiels chevauche la zone aveugle.

Il existe donc un besoin pour un procédé de contrôle non destructif ne présentant pas ces inconvénients, et en particulier adapté à une pièce réfractaire d'épaisseur inférieure à 200 millimètres.

Un but de l'invention est de répondre, au moins partiellement, à ce besoin.

### Résumé de l'invention

L'invention concerne un procédé de contrôle de la structure interne d'une pièce réfractaire, étant en un matériau fondu constitué, pour plus de 90% de sa masse, d'un ou plusieurs oxydes choisis dans le groupe constitué par ZrO₂, Al₂O₃, SiO₂, Cr₂O₃, Y₂O₃, et CeO₂,
ledit procédé comportant les étapes suivantes :
a) envoyer, au moyen d'une antenne émettrice, au moins une onde électromagnétique, dite « impulsion » (« impulsion incidente »), dans la pièce réfractaire à contrôler ;
b) recevoir, au moyen d'une antenne réceptrice, ladite impulsion après qu'elle a été réfléchie par une zone réfléchissante de la pièce réfractaire (« impulsion réfléchie ») ;
c) analyser le décalage temporel entre les deux étapes précédentes afin d'en déduire la position, dans la pièce réfractaire, de la zone réfléchissante,
ladite impulsion ayant une durée inférieure ou égale à 0,5 nanoseconde.

Les inventeurs ont constaté qu'une telle durée permet avantageusement d'évaluer la structure interne de la pièce réfractaire examinée, même si cette pièce réfractaire présente une épaisseur inférieure à 200 millimètres.

De préférence, un procédé selon l'invention présente encore une ou plusieurs des caractéristiques optionnelles suivantes :
- ladite durée est inférieure à 0,3 ns, de préférence inférieure à 0,2 ns, voire inférieure à 0,15 ns ou à 0,10 ns ;
- à l'étape a), on envoie plus de 1000 impulsions par seconde ;
- l'intervalle temporel entre l'émission de deux impulsions est supérieur à 100 ns;
- l'impulsion est un train d'ondes dont la fréquence centrale est de préférence comprise entre 2 et 10 GHz, de préférence supérieure à 3 GHz ;
- l'antenne émettrice et l'antenne réceptrice sont situées à moins de 20 centimètres l'une de l'autre, voire à moins de 10 centimètres, voire à moins de 5 centimètres ;
- la pièce réfractaire présente une épaisseur inférieure à 300 millimètres, inférieure à 200 millimètres, voire inférieure à 100 millimètres ;

Le procédé est de préférence utilisé pour contrôler la structure interne d'une région de la pièce réfractaire qui s'étend, à partir d'une surface de ladite pièce réfractaire, sur une profondeur inférieure à 200 mm.

L'invention concerne également un procédé de fabrication d'un four, en particulier d'un four de verrerie ou d'un four métallurgique, ledit procédé comportant les étapes suivantes :
A) Fabrication d'une pièce réfractaire étant en un matériau fondu constitué, pour plus de 90% de sa masse, d'un ou plusieurs oxydes choisis dans le groupe constitué par ZrO2, Al2O3, SiO2, Cr2O3, Y2O3, et CeO2 ;
B) Contrôle de la pièce réfractaire suivant un procédé de contrôle selon l'invention ;
C) Si le contrôle est satisfaisant, disposition de la pièce réfractaire dans le four, en particulier dans une région où elle est susceptible d'entrer en contact avec un verre ou un métal en fusion.

### Définitions

- L'épaisseur d'une pièce réfractaire est ici définie comme sa plus petite dimension.
- Par "comprenant un" ou "comportant un", on entend "comportant au moins un", sauf indication contraire.

### Description détaillée

Un procédé selon l'invention met en oeuvre les principes bien connus du radar à impulsions. Un radar dit « à impulsions », ou *« pulse radar»* en anglais, doit être distingué d'un radar à onde continue ou à émission continue, en anglais *« continuous wave radar ».* Les principes de fonctionnement de ces radars sont en effet très différents : En particulier, un radar à impulsions émet une impulsion et attend son retour. Au contraire, un radar à émission continue émet continuellement une onde à partir d'une première antenne et reçoit continuellement l'onde réfléchie à l'aide d'une seconde antenne.

De préférence, le procédé selon l'invention met en oeuvre la technologie RPS, en particulier la technologie décrite dans FR 2 689 245, seulement adaptée pour modifier la nature des ondes électromagnétiques émises dans la pièce réfractaire. En particulier, selon l'invention, il est essentiel que les impulsions aient une durée inférieure ou égale à 0,5 nanoseconde, alors que FR 2 689 245 n'évoque que des impulsions ayant une largeur de 1 nanoseconde.

Une impulsion peut être constituée par un signal de forme quelconque, de préférence de forme sinusoïdale, triangulaire, carrée, rectangulaire ou en dent de scie. De préférence l'impulsion est un train d'ondes périodiques qui présentent une fréquence centrale supérieure à 3 Giga Hertz, ce qui améliore la résolution (c'est-à-dire la distance minimale entre deux points pour pouvoir les distinguer séparément). L'impulsion peut être également une onde non périodique, par exemple une impulsion de Dirac.

Les impulsions sont de préférence émises à intervalle régulier. De préférence, on émet plus de 1000 impulsions par seconde. Cette fréquence d'émission des impulsions ne doit pas être confondue avec la fréquence de l'onde qui constitue une impulsion, par exemple de 3 GHz.

La durée, ou « largeur », des impulsions ne doit pas non plus être confondue avec la période de l'onde qui constitue une impulsion. En particulier, c'est le mérite des inventeurs d'avoir découvert que, pour une même période de l'onde, une largeur d'impulsion réduite permet d'améliorer l'analyse de la région superficielle d'une pièce réfractaire, et en particulier de la région de la pièce réfractaire qui s'étend, à partir d'une surface de ladite pièce réfractaire, sur une profondeur inférieure à 200 mm.

De préférence, des impulsions sont émises successivement à partir de différents endroits de la pièce réfractaire à contrôler. De préférence, l'antenne émettrice parcourt une surface extérieure de la pièce réfractaire, par exemple une des faces latérales de la pièce réfractaire. On peut ainsi obtenir une image de la structure interne de la pièce réfractaire.

Lorsque les propriétés du milieu de propagation des ondes électromagnétiques changent, et en particulier en cas d'évolution de la constante diélectrique de ce milieu, une partie de ces ondes est réfléchie. En particulier, des cavités à l'intérieur de la pièce réfractaire peuvent réfléchir les impulsions incidentes. Les impulsions réfléchies peuvent être enregistrées par l'antenne réceptrice.

Les antennes émettrice et réceptrice peuvent être identiques ou différentes.

Dans un mode de réalisation préféré, l'antenne émettrice et l'antenne réceptrice sont disposées côte à côte. Par « côte à côte », on signifie que l'antenne émettrice et l'antenne réceptrice sont en contact l'une avec l'autre ou sont écartées l'une de l'autre d'une distance inférieure à 5 centimètres. La compacité de l'appareil de mesure en est améliorée.

Les antennes émettrice et réceptrice peuvent être avantageusement disposées au même endroit, c'est-à-dire que, pour parvenir à l'antenne réceptrice, l'impulsion réfléchie traverse la zone traversée par l'impulsion incidente immédiatement après qu'elle a été émise par l'antenne émettrice. Les antennes émettrice et réceptrice peuvent être également disposées à des endroits différents.

De préférence, les antennes émettrice et réceptrice sont disposées en contact avec une surface extérieure de la pièce réfractaire à contrôler. De préférence l'antenne émettrice émet perpendiculairement à ladite surface extérieure de la pièce réfractaire et, de préférence encore, lorsque la pièce réfractaire présente un plan de symétrie, perpendiculairement audit plan de symétrie.

A l'étape c), le décalage temporel permet de connaître la position de la zone de la pièce réfractaire qui a réfléchi l'impulsion incidente, la vitesse de l'impulsion dans la pièce réfractaire étant facilement déterminée. Si cette position correspond à une surface extérieure de la pièce réfractaire, aucun défaut n'a été détecté. Sinon, la pièce réfractaire présente une hétérogénéité de structure qui peut être assimilée à un défaut.

L'analyse effectuée à l'étape c) permet d'évaluer la structure interne de la pièce réfractaire contrôlée. De préférence, l'étape c) conduit à l'établissement de représentations de cette structure interne, et en particulier à des images.

Pour les régions profondes de la pièce réfractaire, la précision de l'information obtenue par l'analyse de l'étape c) est similaire à celle obtenue avec des radars à impulsions conventionnels. En revanche, l'étape c) fournit de l'information fiable pour les régions superficielles, à la différence de celle obtenue avec les radars à impulsions conventionnels.

Si un défaut rédhibitoire est identifié, la pièce réfractaire peut être écartée, par exemple pour être mise au rebut ou être recyclée. Sinon, elle peut être exploitée, par exemple disposée dans un four de verrerie, notamment dans une région critique.

### Exemples

Les exemples suivants sont fournis à des fins illustratives et ne limitent pas l'invention.

Un bloc de dimensions 250 mm x 500 mm x 1200 mm et une dalle de dimensions 50 mm x 500 mm x 600 mm, obtenus par un procédé de fusion des matières premières dans un four à arc, puis coulage en moule et solidification, ont été analysés au moyen d'un procédé selon la technologie RPS, en utilisant deux durées d'impulsion de 1 ns (« méthode A »), et de 0,1 ns, conformément à l'invention (« méthode B »). La méthode A est représentative de l'état de la technique.

Dans chacune des deux méthodes, des ondes électromagnétiques sont émises par un radar à impulsions : un radar StructureScan Mini^{™} de la société GSSI pour la méthode A et le radar Groundvue 5C de la société Utsi Electronics pour la méthode B. Les ondes sont des signaux carrés non parfaits. Chaque impulsion est un train d'onde constitué d'une multitude d'ondes sinusoïdales dont les fréquences peuvent varier de plus ou moins 60 à 70% autour de la fréquence centrale, par exemple comprise entre 2 et 10 GHz pour la méthode B. La durée et la fréquence centrale des impulsions sont données dans les tableaux 1 et 2.

Le bloc et la dalle sont posés sur leur face principale (respectivement la face de dimension 500 mm x 1200 mm et la face de dimension 500 mm x 600 mm) et le radar est déplacé le long de la surface opposée. Le bloc et la dalle sont ensuite sciés selon le plan de symétrie transversal de leur face principale pour permettre une observation visuelle des défauts et confronter cette observation aux images obtenues avec les deux méthodes.

Les tableaux 1 et 2 présentent le résultat des observations.

**Tableau 1**

| | Durée d'impulsion | Fréquence centrale | Observation pour le bloc |
|---|---|---|---|
| Méthode A | 1 ns | 1,6 GHz | 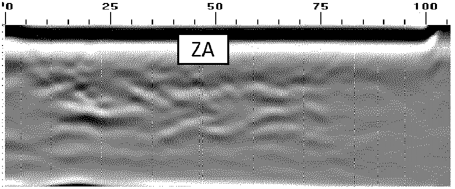 |
| Observation visuelle | | | 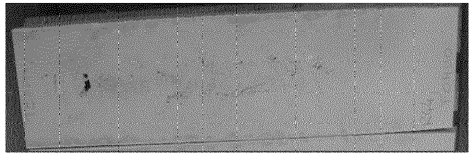 |

**Tableau 2**

| | Durée d'impulsion | Fréquence | Observation pour la dalle |
|---|---|---|---|
| Méthode A | 1 ns | 1,6 GHz | 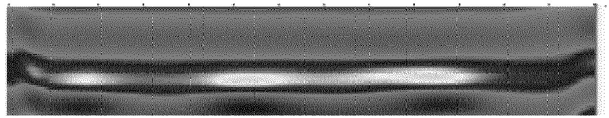 |
| Méthode B | 0,1 ns | 6,0 GHz | 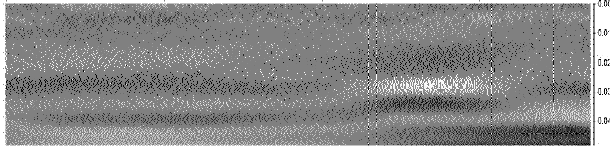 |
| Observation visuelle | | | 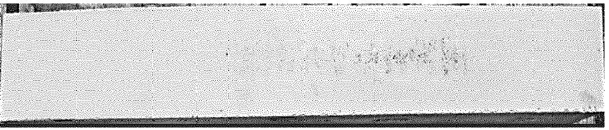 |

On constate que l'utilisation du radar à 1,6GHz et à une nanoseconde de durée d'impulsion laisse une zone aveugle ZA sur le bord de l'image du bloc et est inefficace pour la dalle. En particulier, la zone de défauts (points noirs correspondant à des porosités sur l'observation visuelle) n'apparaît pas du tout sur l'image de la dalle.

Au contraire, avec la méthode B, on voit apparaître des zones blanches qui correspondent à la zone de défauts. On peut noter par ailleurs que la résolution de l'image est très bonne selon cette méthode.

Comme cela apparaît clairement à présent, l'invention fournit un procédé simple à mettre en oeuvre, et en particulier qui ne nécessite pas d'immersion de la pièce réfractaire à contrôler, et efficace sur toute l'épaisseur de la pièce réfractaire. Ce procédé est particulièrement adapté au contrôle de pièces réfractaires de faible épaisseur comme des dalles.

Bien entendu, l'invention n'est pas limitée aux modes de réalisation décrits, fournis à titre illustratif et non limitatif.

## Revendications

1. Procédé de contrôle de la structure interne d'une pièce réfractaire étant en un matériau fondu constitué, pour plus de 90% de sa masse, d'un ou plusieurs oxydes choisis dans le groupe constitué par ZrO₂, Al₂O₃, SiO₂, Cr₂O₃, Y₂O₃, et CeO₂, ledit procédé comportant les étapes suivantes :
a) envoyer, au moyen d'une antenne émettrice, au moins une onde électromagnétique, dite « impulsion », dans la pièce réfractaire à contrôler ;
b) recevoir, au moyen d'une antenne réceptrice, ladite impulsion après qu'elle a été réfléchie par une zone réfléchissante de la pièce réfractaire ;
c) analyser le décalage temporel entre les deux étapes précédentes afin d'en déduire la position, dans la pièce réfractaire, de la zone réfléchissante,
ladite impulsion ayant une durée inférieure ou égale à 0,5 nanoseconde.

2. Procédé selon la revendication précédente, dans lequel la durée est inférieure à 0,10 nanoseconde.

3. Procédé selon l'une quelconque des revendications précédentes, dans lequel l'impulsion est un train d'ondes qui présente une fréquence centrale supérieure à 3 Giga Hertz.

4. Procédé selon l'une quelconque des revendications précédentes, dans lequel, à l'étape a), on envoie plus de 1000 impulsions par seconde et/ou dans lequel deux impulsions successives sont séparées de plus de 100 ns.

5. Procédé selon l'une quelconque des revendications précédentes, dans lequel l'antenne émettrice et l'antenne réceptrice sont côte à côte.

6. Procédé selon l'une quelconque des revendications précédentes, dans lequel la pièce réfractaire présente une épaisseur inférieure à 300 millimètres.

7. Procédé selon la revendication précédente, dans lequel la pièce réfractaire présente une épaisseur inférieure à 200 millimètres.

8. Procédé de fabrication d'un four, en particulier d'un four de verrerie ou d'un four métallurgique, ledit procédé comportant les étapes suivantes :
A) Fabrication d'une pièce réfractaire étant en un matériau fondu et étant constitué, pour plus de 90% de sa masse, d'un ou plusieurs oxydes choisis dans le groupe constitué par ZrO₂, Al₂O₃, SiO₂, Cr₂O₃, Y₂O₃, et CeO₂;
B) Contrôle de la pièce réfractaire suivant un procédé de contrôle selon l'une quelconque des revendications précédentes ;
C) Si le contrôle est satisfaisant, disposition de la pièce réfractaire dans le four, en particulier dans une région où elle est susceptible d'entrer en contact avec un verre ou un métal en fusion.

## Patentansprüche

1. Verfahren zur Überprüfung der inneren Struktur eines hitzebeständigen Werkstücks, das aus einem geschmolzenen Material ist, welches zu mehr als 90 % seiner Masse aus einem oder mehreren Oxiden besteht, die aus der Gruppe ausgewählt sind, welche aus ZrO₂, Al₂O₃, SiO₂, Cr₂O₃, Y₂O₃ und CeO₂ besteht, wobei das Verfahren die folgenden Schritte umfasst:
a) Aussenden, mittels einer Sendeantenne, mindestens einer elektromagnetischen Welle, die als "Impuls" bezeichnet wird, in das zu prüfende hitzebeständige Werkstück;
b) Empfangen, mittels einer Empfangsantenne, des Impulses nachdem dieser von einem reflektierenden Bereich des hitzebeständigen Werkstücks reflektiert wurde;
c) Auswerten des Zeitversatzes zwischen den beiden vorhergehenden Schritten, um daraus die Position des reflektierenden Bereichs innerhalb des hitzebeständigen Werkstücks abzuleiten,
wobei der Impuls eine Dauer von höchstens 0,5 Nanosekunden hat.

2. Verfahren nach dem vorhergehenden Anspruch, wobei die Dauer weniger als 0,10 Nanosekunden beträgt.

3. Verfahren nach einem beliebigen der vorhergehenden Ansprüche, wobei es sich bei dem Impuls um ein Wellenpaket handelt, das eine Mittelfrequenz von mehr als 3 Gigahertz aufweist.

4. Verfahren nach einem beliebigen der vorhergehenden Ansprüche, wobei im Schritt a) mehr als 1000 Impulse pro Sekunde ausgesendet werden und/oder wobei zwei aufeinanderfolgende Impulse um mehr als 100 ns beabstandet sind.

5. Verfahren nach einem beliebigen der vorhergehenden Ansprüche, wobei die Sendeantenne und die Empfangsantenne sich nebeneinander befinden.

6. Verfahren nach einem beliebigen der vorhergehenden Ansprüche, wobei das hitzebeständige Werkstück eine Dicke von weniger als 300 Millimetern aufweist.

7. Verfahren nach dem vorhergehenden Anspruch, wobei das hitzebeständige Werkstück eine Dicke von weniger als 200 Millimetern aufweist.

8. Verfahren zur Herstellung eines Ofens, insbesondere eines Glasschmelzofens oder eines metallurgischen Ofens, wobei das Verfahren die folgenden Schritte aufweist:
A) Herstellen eines hitzebeständigen Werkstücks, das aus einem geschmolzenen Material ist und das zu mehr als 90 % seiner Masse aus einem oder mehreren Oxiden besteht, die aus der Gruppe ausgewählt sind, welche aus ZrO₂, Al₂O₃, SiO₂, Cr₂O₃, Y₂O₃ und CeO₂ besteht;
B) Überprüfen des hitzebeständigen Werkstücks gemäß einem Überprüfungsverfahren nach einem beliebigen der vorhergehenden Ansprüche;
C) wenn die Überprüfung zufriedenstellend ausfällt, Anordnen des hitzebeständigen Werkstücks in dem Ofen, insbesondere in einer Region, wo dieses mit einer Glas- oder Metallschmelze in Kontakt kommen kann.

## Claims

1. Method for inspecting the internal structure of a refractory part made of a fused material that consists, for more than 90% of its weight, of one or more oxides chosen from the group made up of ZrO₂, Al₂O₃, SiO₂, Cr₂O₃, Y₂O₃, and CeO₂, said method including the following steps:
a) transmitting, by means of an emitting antenna, at least one electromagnetic wave, called the "pulse", into the refractory part to be inspected;
b) receiving, by means of a receiving antenna, said pulse after it has been reflected by a reflecting zone of the refractory part; and
c) analysing the time shift between the two preceding steps in order to deduce therefrom the position, in the refractory part, of the reflecting zone,
said pulse having a duration shorter than or equal to 0.5 nanoseconds.

2. Method according to the preceding claim, wherein the duration is shorter than 0.10 nanoseconds.

3. Method according to any one of the preceding claims, wherein the pulse is a wave train that has a central frequency higher than 3 gigahertz.

4. Method according to any one of the preceding claims, wherein, in step a), more than 1000 pulses per second are transmitted and/or wherein two successive pulses are separated by more than 100 ns.

5. Method according to any one of the preceding claims, wherein the emitting antenna and the receiving antenna are side-by-side.

6. Method according to any one of the preceding claims, wherein the refractory part has a thickness smaller than 300 millimetres.

7. Method according to the preceding claim, wherein the refractory part has a thickness smaller than 200 millimetres.

8. Method for manufacturing a furnace, in particular a glass furnace or a metallurgical furnace, said method including the following steps:
A) Manufacturing a refractory part made of a fused material that consists, for more than 90% of its weight, of one or more oxides chosen from the group made up of ZrO₂, Al₂O₃, SiO₂, Cr₂O₃, Y₂O₃, and CeO₂;
B) Inspecting the refractory part using an inspecting method according to any one of the preceding claims; and
C) If the inspection is passed, placing the refractory part in the furnace, in particular in a region in which it is liable to make contact with molten metal or glass.
